(19) **Europäisches Patentamt European Patent Office Office européen des brevets**

(11) **EP 4 447 069 A1**

(12) **EUROPEAN PATENT APPLICATION**
published in accordance with Art. 153(4) EPC

(43) Date of publication:
**16.10.2024 Bulletin 2024/42**

(21) Application number: **22904598.4**

(22) Date of filing: **05.12.2022**

(51) International Patent Classification (IPC):
**G16H 50/50** (2018.01)     **G16H 20/40** (2018.01)
**G16H 50/20** (2018.01)     **G16B 30/10** (2019.01)
**G16B 50/00** (2019.01)     **G16H 30/40** (2018.01)
**C12Q 1/6886** (2018.01)     **G01N 33/483** (2006.01)
**G06T 7/00** (2017.01)     **G06F 17/18** (2006.01)

(52) Cooperative Patent Classification (CPC):
**C12Q 1/6886; G01N 33/483; G06F 17/18;
G06T 7/00; G16B 30/10; G16B 50/00; G16H 20/40;
G16H 30/40; G16H 50/20; G16H 50/50**

(86) International application number:
**PCT/KR2022/019625**

(87) International publication number:
**WO 2023/106768 (15.06.2023 Gazette 2023/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.12.2021 KR 20210172562**

(71) Applicant: **GC Genome Corporation
Yongin-si, Gyeonggi-do 16924 (KR)**

(72) Inventors:
• **CHO, Eun-Hae
Yongin-si Gyeonggi-do 16924 (KR)**

• **AHN, Jin Mo
Yongin-si Gyeonggi-do 16924 (KR)**
• **LEE, Junnam
Yongin-si Gyeonggi-do 16924 (KR)**
• **LEE, Tae-Rim
Yongin-si Gyeonggi-do 16924 (KR)**
• **SOHN, Joohyuk
Seoul 06630 (KR)**
• **KIM, Gun Min
Seoul 03722 (KR)**
• **KIM, Min Hwan
Goyang-si Gyeonggi-do 10496 (KR)**

(74) Representative: **Mewburn Ellis LLP
Aurora Building
Counterslip
Bristol BS1 6BX (GB)**

(54) **BLOOD CELL-FREE DNA-BASED METHOD FOR PREDICTING PROGNOSIS OF BREAST CANCER TREATMENT**

(57) The present invention relates to a blood cell-free DNA-based method for predicting prognosis of breast cancer treatment and, more particularly, to a cell-free DNA-based method for predicting prognosis of breast cancer treatment, the method comprising a step of extracting cell-free DNA (cfDNA) from a biological sample before anticancer treatment, acquiring sequence information, then obtaining an I-score by using normalization correction and regression analysis of chromosomal regions, and analyzing the I-score and image information of the breast together after the anticancer treatment. A method for predicting prognosis of breast cancer, according to the present invention, uses next generation sequencing (NGS) so as to increase the accuracy of predicting the prognosis of a breast cancer patient and also increase the accuracy of prognosis prediction based on a very low concentration cell-free DNA of which detection has been difficult, thereby increasing the commercial utilization thereof. Therefore, the method of the present invention is useful in determining the prognosis of a breast cancer patient.

EP 4 447 069 A1

FIG. 1

Extracting cell-free nucleic acid from plasma

↓

Obtaining nucleic acid fragments by massive parallel sequencing

↓

Aligning fragment data to human reference chromosomes

↓

Checking quality of aligned data

↓

Calculating I-score

↓

Determining prognosis using I-score and pCR

**Description**

[Technical Field]

The present invention relates to a method for predicting the prognosis of breast cancer treatment based on blood cell-free DNA and more specifically to a method for predicting the prognosis of breast cancer treatment by extracting cell-free DNA (cfDNA) from a biological sample before chemotherapy to obtain sequence information, performing normalization and regression analysis in the chromosomal region to obtain an I score, and analyzing the I score and image information of the breast after chemotherapy.

[Background Art]

Breast cancer, which is a mass composed of cancer cells grown in the breast, is known to be the second most common type of cancer worldwide after lung cancer and is the cancer with the fifth highest mortality rate after lung cancer, stomach cancer, breast cancer, and colon cancer, and is the most common cancer in women and the second highest mortality rate.
Risk factors for developing breast cancer include race, age, mutations in the cancer suppressor genes BRCA-1 and BRCA-2 and p53, and the like. Alcohols, high-fat diet, lack of exercise, exogenous postmenopausal hormones, and ionizing radiation also increase the risk of developing breast cancer. Breast cancer is divided into four subtypes: luminal A breast cancer, luminal B breast cancer, HER2 breast cancer, and triple negative breast cancer (TNBC), depending on the expression status of hormone receptors (estrogen receptors or progesterone receptors) and HER2 (human epidermal growth factor receptor 2). Each breast cancer subtype has distinguishing molecular characteristics.
Additional auxiliary treatment such as chemotherapy, anti-hormone treatment, targeted treatment, or radiation therapy after tumor removal surgery, as current treatment methods for breast cancer, may be required to reduce future recurrence. Although 70 to 80% of early-stage breast cancer patients required no chemotherapy due to very low risk of metastasis to other organs, the majority of patients receive chemotherapy and radiation treatment after surgery because accurate determination is difficult using conventional breast cancer treatment guidelines. However, continuously administering anticancer drugs to patients for whom the effects of chemotherapy will not be great may increase side effects and cause undesired pain to the patients. Therefore, it is necessary to clearly predict the future cancer prognosis in early-stage breast cancer patients, wisely select the most appropriate treatment method at the present time, and prepare for bad prognosis such as metastatic recurrence.
Once breast cancer treatment begins, the progression of cancer must be monitored periodically. However, cost and time are required depending on the diagnosis method, and it is very difficult to detect and diagnose cancer when the patient tumor is small or the number of cancer cells is small. Although there are some products for predicting prognosis, they are still expensive, cannot detect the condition during the treatment process and predict simple prognosis at a single time of examination.
Meanwhile, the focus has been on proliferation and cell cycle signals as prognostic indicators of breast cancer, and thus proliferation/cell cycle regulatory genes have been used as markers for gene expression-based analysis to predict prognosis. Representative products such as Oncotype DX, MammaPrint, PAM50, and Endopredict are used for commercial analysis based on multiplex gene expression profiling techniques targeting proliferative genes in frozen or formalin-fixed paraffin-embedded (FFPE) samples. However, these commercial kits each have a limitation in the breast cancer subtypes they target and thus are widely inapplicable to all breast cancer molecular subtypes. The Oncotype DX, MammaPrint, PAM50, and Endopredict kits mainly target ER+ type breast cancer. These commercial kits are only capable of predicting prognosis for hormone receptor-positive breast cancer subtypes and thus commercial kits for hormone receptor-negative breast cancer subtypes have been not developed yet.
Considering the current situation, there is need for development of conventional analysis methods used to predict breast cancer prognosis in order to more accurately predict survival outcomes of patients and response of patients to adjuvant chemotherapy and for prognostic analysis methods applicable to various types of breast cancer.
Furthermore, recently, studies have been conducted to detect chromosomal abnormalities using cell-free DNA (cfDNA), which is present in plasma through necrosis, apoptosis and secretion of cells, based on liquid biopsy technology. In particular, blood-cell-free DNA derived from tumor cells includes tumor-specific chromosomal abnormalities and mutations that are not found in normal cells, and has the advantage of reflecting the current state of tumors due to the short half-life thereof of 2 hours. In addition, blood-cell-free DNA is in the spotlight as a tumor-specific biomarker in various cancer-related fields such as diagnosis, monitoring and prognosis of cancer because collection thereof is noninvasive and can be performed repeatedly.
With recent advances in molecular diagnostic technology, research has reported that it is possible to detect tumor-specific chromosomal abnormalities in blood-cell-free DNA of cancer patients through digital karyotyping and PARE analysis, and the results of research have clinically confirmed the same (Leary R. J. et al., Sci. Transl. Med. Vol.

4, Issue 162. 2012). Daniel G. Stover analyzed tissue-specific CNA through cfDNA in 164 metastatic TNBC (triple-negative breast cancer) patients (Stover DG. et al., J. Clin. Oncol. Vol. 36(6):543-553). The result showed that the increase in the number of copies of specific genes such as NOTCH2, AKT2 and AKT3 was higher in metastatic TNBC than in primary TNBC, and the survival rate of metastatic TNBC patients with overlapping 18q11 and 19p13 chromosomes was statistically significantly lower.

Accordingly, against this technical background, as a result of extensive efforts to develop a method for determining the prognosis of breast cancer based on cell-free DNA in the blood, the present inventors found that when performing normalization correction and regression analysis in blood-cell-free DNA in a blood sample obtained before chemotherapy and the results are analyzed in combination with video image reading information after chemotherapy, the prognosis of breast cancer patients can be determined with high sensitivity. Based on this finding, the present invention was completed.

[Disclosure]

Therefore, the present invention has been made in view of the above problems, and it is one object of the present invention to provide a method of determining the prognosis of breast cancer based on cell-free DNA (cfDNA).

It is another object of the present invention to provide a device for determining the prognosis of breast cancer.

It is another object of the present invention to provide a computer-readable medium including instructions designed to be executed by a processor for determining the prognosis of breast cancer using the method.

It is another object of the present invention to provide a method of providing information for determining the prognosis of breast cancer including the method.

It is another object of the present invention to provide a method of determining the prognosis of breast cancer including the method.

In accordance with one aspect of the present invention, the above and other objects can be accomplished by the provision of a method of predicting a prognosis of breast cancer based on cell-free DNA (cfDNA), the method including: a) obtaining reads (sequence information) of the cell-free DNA isolated from a biological sample before chemotherapy; b) aligning the reads to a reference genome database of a reference group; c) detecting a quality of the aligned reads and selecting only reads having a quality equal to or higher than a cut-off value; d) segmenting the reference genome into predetermined bins, and detecting and normalizing amounts of the selected reads in the respective bins; e) calculating a mean and a standard deviation of normalized reads matched to each bin of the reference group and then calculating a Z score from normalized values in step d); f) segmenting chromosome using the Z score and calculating an I score; g) obtaining breast tissue image reading information after chemotherapy; and h) determining that a breast cancer prognosis is bad when the resulting I score is equal to or higher than a cut-off value and the read breast tissue image information is positive.

In accordance with another aspect of the present invention, provided is a device for predicting a prognosis of breast cancer based on cell-free DNA (cfDNA), the device including: a decoder for decoding reads (sequence information) of cell-free DNA isolated from a biological sample before chemotherapy; an aligner for aligning the decoded reads to a reference genome database of a reference group; a quality controller for selecting only reads having a quality equal to or higher than a cut-off value from the aligned reads; an I score calculator for calculating a Z score of the selected sequence information (reads) by comparison with a reference group sample and then calculating an I score (I-score) based thereon; a read image information receiver for obtaining breast tissue image reading information after chemotherapy; and a determiner for determining that the prognosis of breast cancer is bad when the I score is equal to or higher than a cut-off value and the read image information is positive.

In accordance with another aspect of the present invention, provided is a computer-readable medium including an instruction configured to be executed by a processor for determining a prognosis of breast cancer, the computer-readable medium including: a) obtaining reads (sequence information) of cell-free DNA isolated from a biological sample before chemotherapy; b) aligning the reads to a reference genome database of a reference group; c) detecting a quality of the aligned reads and selecting only reads having a quality equal to or higher than a cut-off value; d) segmenting the reference genome into predetermined bins, and detecting and normalizing amounts of the selected reads in the respective bins; e) calculating a mean and a standard deviation of normalized reads matched to each bin of the reference group and then calculating a Z score from normalized values in step d); f) segmenting chromosome using the Z score and calculating an I score; g) obtaining breast tissue image reading information after chemotherapy; and h) determining that a prognosis of breast cancer is bad when the resulting I score is equal to or higher than a cut-off value and the read breast tissue image information is positive.

In accordance with another aspect of the present invention, provided is a method of providing information for determining the prognosis of breast cancer including the method.

In accordance with another aspect of the present invention, provided is a method of determining the prognosis of breast cancer including the method.

[Description of Drawings]

FIG. 1 is an overall flowchart showing the determination of prognosis of breast cancer based on cfDNA according to the present invention.

FIG. 2 is a schematic diagram showing the result of calibration of the number of sequencing reads before and after GC calibration using a LOESS algorithm during the process of quality control (QC) of read data.

FIG. 3 shows the results of Kaplan Meier analysis to predict the progression and survival probability of breast cancer according to the method of the present invention, wherein (A) shows the result from the exploratory group and (B) shows the result from the validation group.

FIG. 4 shows the results of hazard analysis to predict the progression and survival probability of breast cancer according to the method of the present invention, wherein (A) shows the result from the exploratory group and (B) shows the result from the validation group.

FIG. 5 shows the results of Kaplan Meier analysis to determine the relationship between I score and pathological complete response (pCR), wherein (A) shows the result from the exploratory group and (B) shows the result from the validation group.

FIG. 6 shows the result of prediction of prognosis on the survival probability of breast cancer patients in each of groups classified on the basis of an I score and pCR according to the present invention.

FIG. 7 shows the result of determination of hazard on the prognosis on survival of breast cancer in each of groups classified on the basis of an I score and pCR according to the present invention.

[Best Mode]

Unless defined otherwise, all technical and scientific terms used herein have the same meanings as appreciated by those skilled in the field to which the present invention pertains. In general, the nomenclature used herein is well-known in the art and is ordinarily used.

Terms such as first, second, A, B, and the like may be used to describe various elements, but these elements are not limited by these terms and are merely used to distinguish one element from another. For example, without departing from the scope of the technology described below, a first element may be referred to as a second element and in a similar way, the second element may be referred to as a first element. "And/or" includes any combination of a plurality of related recited items or any one of a plurality of related recited items.

Singular forms are intended to include plural forms as well, unless the context clearly indicates otherwise. It will be further understood that the terms "comprises" and/or "comprising", when used in this specification, specify the presence of features, numbers, steps, actions, components, parts, or combinations thereof, but do not preclude the presence or addition of one or more other features, numbers, steps, actions, components, parts, or combinations thereof.

Prior to the detailed description of the drawings, it is to be clarified that the classification of components in the present specification is merely made depending on the main function of each component. That is, two or more components described below may be combined into one component or one component may be divided into two or more depending on each more detailed function. In addition, each component to be described below may further perform some or all of the functions of other components in addition to its main function, and some of the main functions of each component may be performed exclusively by other components.

In addition, in implementing a method or operation method, respective steps constituting the method may occur in a different order from a specific order unless the specific order is clearly described in context. That is, the steps may be performed in the specific order, substantially simultaneously, or in reverse order to that specified.

It was found in the present invention that sequence analysis data (reads) obtained from a breast cancer patient sample was normalized and organized based on a cut-off value, the amount of reads in each bin was normalized, a Z score was calculated through comparison with a reference group sample, chromosome was segmented again based on the calculated Z score, an I score was calculated based thereon, and the prognosis was determined to be bad when the I-score was greater than or equal to a cut-off value, and was determined to be good when the I-score was less than the cut-off value. Specifically, the risk groups for death from breast cancer or progression thereof could be classified and identified depending on the cut-off value of the I score and the presence of pathological complete response (pCR).

More specifically, the case where the I score is lower than a cut-off value and the read image information is positive is classified as a moderate-risk group, the case where the I score is lower than a cut-off value, and the read image information is positive, is classified as a high-risk group, and the case where the I score is equal to or higher than a cut-off value and the read image information is positive is classified as an ultra-high-risk group.

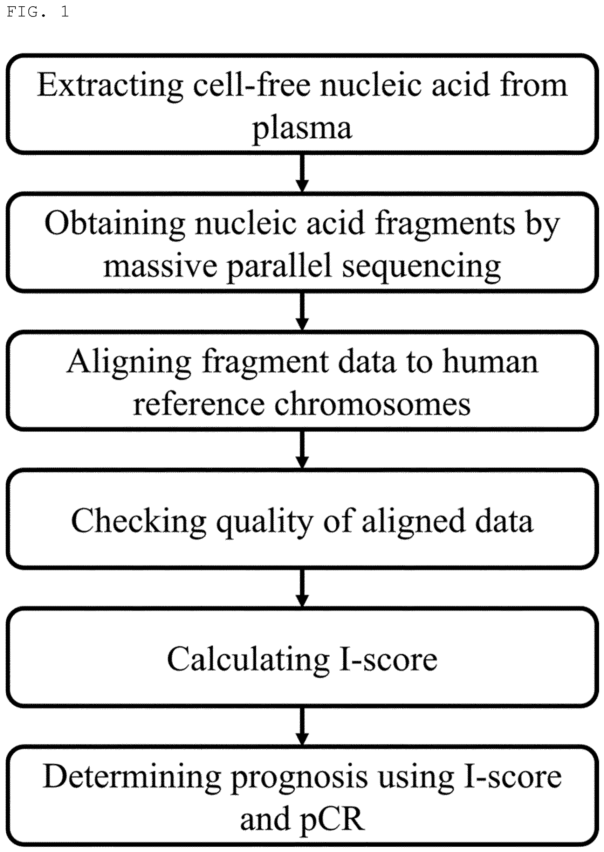

That is, in an embodiment of the present invention, developed was a method of determining the prognosis of breast cancer including sequencing DNA extracted from the blood of 20 normal subjects and 456 breast cancer patients before chemotherapy, controlling quality using the LOESS algorithm, segmenting chromosome into predetermined bins to normalize the amount of reads matched to each bin with a GC ratio, calculating the mean and standard deviation of the reads matched to each bin in a normal sample, calculating a Z score with the normalized value, segmenting an area of a chromosome where the Z score rapidly changes again based thereon, calculating an I-score using the same, analyzing pCR information of breast cancer patients before chemotherapy, and determining that the prognosis of the breast cancer patients is bad when the I-score is equal to or higher than 7.81 and pCR is negative (FIG. 1).

As used herein, the term "read" refers to one nucleic acid fragment obtained by analyzing sequence information using any of a variety of methods known in the art. Therefore, the term "read" has the same meaning as the term "sequence information" in that they both refer to sequence information results obtained through a sequencing process.

As used herein, the term "prediction of prognosis" has the same meaning as the term "prognosis", and refers to an act of predicting the course and outcome of a disease in advance. More specifically, the term "prediction of prognosis" is interpreted to mean any action that predicts the course of a disease after treatment in comprehensive consideration of the physiological or environmental state of a patient, and the course of the disease after treatment of the disease may vary depending on the physiological or environmental state of the patient.

For the purposes of the present invention, the prediction of prognosis can be interpreted as an act of predicting the progression of a disease after treatment of breast cancer and predicting the risk of progression of cancer, recurrence of cancer, and/or metastasis of cancer. For example, the expression "good prognosis" or "prognosis is good" means that the risk index of progression of cancer, recurrence of cancer and/or metastasis of cancer in a breast cancer patient after breast cancer treatment is lower than 1 and that the breast cancer patient is more likely to survive, and is also expressed as "positive prognosis". The expression "bad prognosis" means that the risk of progression of cancer, recurrence of cancer and/or metastasis of cancer in a breast cancer patient after breast cancer treatment is higher than 1, and that the breast cancer patient is more likely to die, and is also expressed as "negative prognosis".

As used herein, the term "risk index" refers to an odds ratio, a hazard ratio, or the like regarding the probability that progression, recurrence, and/or metastasis of cancer will occur in a patient after treatment of breast cancer.

In one aspect, the present invention is directed to a method of predicting a prognosis of breast cancer based on cell-free DNA (cfDNA), the method including:

a) obtaining reads (sequence information) of the cell-free DNA isolated from a biological sample before chemotherapy;

b) aligning the reads to a reference genome database of a reference group;

c) detecting a quality of the aligned reads and selecting only reads having a quality equal to or higher than a cut-off value;

d) segmenting the reference genome into predetermined bins, and detecting and normalizing amounts of the selected reads in the respective bins;

e) calculating a mean and a standard deviation of normalized reads matched to each bin of the reference group and then calculating a Z score from normalized values in step d) ;

f) segmenting chromosome using the Z score and calculating an I score;

g) obtaining breast tissue image reading information after chemotherapy; and

h) determining that a prognosis of breast cancer is bad when the resulting I score is equal to or higher than a cut-off value and the read breast tissue image information is positive.

In the present invention, anticancer treatment may be used without limitation as long as it is any method that can treat cancer and is preferably selected from the group consisting of neoadjuvant therapy, neoadjuvant chemotherapy, adjuvant chemotherapy, surgery, and radiotherapy, but is not limited thereto.

In the present invention,

step a) is carried out by a process including:

(a-i) removing proteins, fats and other residues from the isolated cell-free DNA using a salting-out method, a column chromatography method, or a bead method to obtain purified nucleic acids;

(a-ii) producing a single-end-sequencing or paired-end-sequencing library from the purified nucleic acids;

(a-iii) applying the produced library to a next-generation sequencer; and

(a-iv) obtaining reads of the nucleic acids from the next-generation sequencer.

The method may further include, between the steps (a-i) and (a-ii), randomly fragmenting the nucleic acids purified in the step (a-i) by an enzymatic digestion, pulverization or HydroShear method to produce the single-end sequencing or paired-end sequencing library.

In the present invention, step a) of obtaining the reads may include obtaining the isolated cell-free DNA through full-length genome sequencing with a depth of 0.01 to 100 reads.

In the present invention, the next-generation sequencer may be a Hiseq system produced by Illumina Inc., a Miseq system produced by Illumina Inc., a genome analyzer (GA) produced by Illumina Inc., 454 FLX produced by Roche Applied Science, SOLiD system produced by Applied Biosystems Company, or the Ion Torrent system produced by Life Technologies Company, but is not limited thereto.

In the present invention, the biological sample refers to any substance, biological fluid, tissue or cell obtained from or derived from a subject, and examples thereof include, but are not limited to, whole blood, leukocytes, peripheral blood mononuclear peripheral cells, leukocyte buffy coat, blood including plasma and serum, sputum, tears, mucus, nasal washes, nasal aspirates, breath, urine, semen, saliva, peritoneal washings, pelvic fluids, cyst fluids, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, semen, hair, saliva, urine, oral cells, placenta cells, cerebrospinal fluid, and mixtures thereof.

As used herein, the term "reference group" refers to a reference group that can be used for comparison, like a standard nucleotide sequence database, and means a population of humans who do not currently have a specific disease or condition. In the present invention, the standard nucleotide sequence in the standard genome database of the reference group may be reference genome registered with a public health institution such as NCBI.

In the present invention, the alignment may be performed using the BWA algorithm and the Hg19 sequence, but is not limited thereto.

In the present invention, the BWA algorithm may include BWA-ALN, BWA-SW, Bowtie2 or the like, but is not limited thereto.

In the present invention, step c) of detecting the quality of the aligned reads means detecting how much the actual read sequence matches the reference genome sequence using a mapping quality score.

In the present invention, step c) is carried out through a process including:

(c-i) specifying a region of each aligned nucleic acid sequence; and

(c-ii) selecting a sequence satisfying a cut-off value of a mapping quality score and a cut-off value of a GC ratio within the region.

In the present invention, in step (c-i) of specifying the region of the nucleic acid sequence, the region of the nucleic acid sequence may have a length of 20 kb to 1 Mb, but is not limited thereto.

In the present invention, in step (c-ii), the cut-off value may vary depending on the desired degree of the mapping quality score, but is specifically 15 to 70, more specifically 30 to 65, and most specifically 60. In step (c-ii), the GC ratio may vary depending on the desired degree of the GC ratio, but is specifically 20 to 70%, and more specifically 30 to 60%.

In the present invention, step c) may be performed excluding data of the centromere or the telomere of the chromosome.

As used herein, the "centromere" may have a length of about 1 Mb from the starting point of each chromosome long arm (q arm), but is not limited thereto.

As used herein, the "telomere" may have a length of about 1 Mb from the starting point of each chromosome short arm (p arm) or about 1 Mb from the ending point of each chromosome long arm (q arm), but is not limited thereto.

In the present invention, step d) is carried out through a process including:

(d-i) segmenting the reference genome into predetermined bins;

(d-ii) calculating the number of reads aligned in each bin and an amount of GC of the reads;

(d-iii) performing a regression analysis based on the number of reads and the amount of GC to calculate a regression coefficient; and

(d-iv) normalizing the number of reads using the regression coefficient.

In the present invention, the predetermined bin in step (d-i) may be 100 kb to 2,000 kb in length.

In the present invention, in step (d-i) of specifying the region of the nucleic acid sequence, the predetermined bin is 100 kb to 2 Mb, specifically 500 kb to 1500 kb, more specifically 600 kb to 1600 kb, more specifically 800 kb to 1200 kb, most specifically 900 kb to 1100 kb, but is not limited thereto.

In the present invention, the regression analysis in step (iii) may be any regression analysis method capable of calculating a regression coefficient, and is specifically LOESS analysis, but is not limited thereto.

In the present invention, step e) of calculating the Z score may include standardizing the sequencing read in each specific bin, and the calculation may be specifically carried out using Equation 1 below.

$$Z\ score = \frac{\text{Read value of sequence information sample of biological specimen} - \text{Mean sequence information read value of reference group}}{\text{Standard deviation of mean sequence information read value of reference group}}$$

[Equation 1]

In the present invention, step (f) includes:

(f-i) segmenting a chromosome region using circular binary segmentation (CBS) based on a Z score in each bin;

(f-ii) obtaining a Z score of each chromosome segment as a mean of Z cores calculated in respective bins included in the segment;

(f-iii) calculating the smoothed Z score (Zn) by performing local regression analysis (LOESS) on each bin,

wherein $n \in \{1,...,N\}$ in which N is the total number of bins;

(f-iv) calculating n_score associated with noise in accordance with the following Equation 2:

$$\text{Equation 2:}\quad n_{score} = mean(|B_{n+1} - B_n|)$$

wherein $B_n$ = *non smoothed binZscore,* which means the Z score of each bin calculated in step i); and

(f-v) calculating an I-score in accordance with the following Equation 3:

$$\text{Equation 3:}\quad \text{Iscore} = \log\{\sum_{n=1}^{N}(|Z_n \times S_n|)\} - n\_score$$

wherein $S_n$ = *segment Zscore of bin*$_n$ which means the Z score of each segment calculated in step i).

In the present invention, the CBS algorithm refers to a method of detecting the point at which a change in the Z score, calculated in the step described above, occurs.

That is, the following Equation is satisfied under the condition of 1<=i<j<=N on the assumption that i is the point at which the change of the Z score of the chromosome begins, j is the point at which the change of the Z score of the chromosome ends, N is the total length of the region, r is the bin value of each nucleic acid sequence (specific bin), and s is a standard deviation of bins.

[Equation 4]

$$S_i = r_1 + r_2 + \cdots + r_i$$

[Equation 5]

$$S_j = r_1 + r_2 + \cdots + r_j$$

[Equation 6]

$$S_{ij} = S_j - S_i = \sum_{n=i+1}^{j} r_n$$

[Equation 7]

$$T_{ij} = \left(\frac{S_{ij}}{j-i} - \frac{S_{j-i}-S_{ij}}{N-j-i}\right) / \left(s\sqrt{\frac{1}{j-i} + \frac{1}{N-j-i}}\right)$$

[Equation 8]

$$(i_c, j_c) = \arg\max|T_{ij}|$$

Here, $(i_c, j_c)$ represents a location at which the Z score change actually occurred, max represents a maximum value, and arg means a declination.

In the present invention, the cut-off value of the I score may be used without limitation as long as it is a value used to predict prognosis, and is preferably 5 to 10, and most preferably 7.81, but is not limited thereto.

In the present invention, the breast tissue image may be used without limitation as long as it enables determination of the presence or absence of cancer cells after chemotherapy. The breast tissue image is preferably a magnetic resonance imaging (MRI) image, a histochemical-stain breast tissue sample image, an ultrasound image, an X-ray image, or a fluorescent stain breast tissue sample image, more preferably, a histochemical-stain breast tissue sample image or a fluorescent stain breast tissue sample image, but is not limited thereto.

In the present invention, the positive breast tissue image reading information means that cancer cells are identified in the image, and negative breast tissue image reading information means that cancer cells are not identified in the image.

In the present invention, the breast tissue image reading information may be used as an indicator for determining pathological complete response. Pathological complete response is defined as the absence of breast invasive cancer in breast cancer patients who receive neoadjuvant therapy and surgery.

In the present invention, the method further may include classifying a case where the I score is equal to or higher than a cut-off value and the read image information is negative as a moderate risk group, classifying a case where the I score is lower than a cut-off value and the read image information is positive, as a high risk group, and classifying a case where the I score is equal to or higher than a cut-off value and the read image information is positive as an ultra-high risk group.

In another aspect, the present invention is directed to a device for predicting a prognosis of breast cancer based on cell-free DNA (cfDNA), the device including: a decoder for decoding reads (sequence information) of cell-free DNA isolated from a biological sample before chemotherapy;

an aligner for aligning the decoded reads to a reference genome database of a reference group;

a quality controller for selecting only reads having a quality equal to or higher than a cut-off value from the aligned reads;

an I score calculator for calculating a Z score of the selected sequence information (reads) by comparison with a reference group sample and then calculating an I score (I-score) based thereon;

a read image information receiver for obtaining breast tissue image reading information after chemotherapy; and

a determiner for determining that the prognosis of breast cancer is bad when the resulting I score is equal to or higher than a cut-off value and the read image information is positive.

In the present invention, the decoder may include a nucleic acid injector configured to inject the nucleic acid extracted from an independent device, and a sequence information analyzer configured to analyze the sequence information of the injected nucleic acid, preferably an NGS analyzer, but is not limited thereto.

In the present invention, the decoder may receive and decode sequence information data generated in an independent device.

In the present invention, the read image information receiver receives read image information generated in the independent device.

In another aspect, the present invention is directed to a computer-readable medium including an instruction configured to be executed by a processor for determining a prognosis of breast cancer, the computer-readable medium including:

a) obtaining reads (sequence information) of cell-free DNA isolated from a biological sample before chemotherapy; b) aligning the reads to a reference genome database of a reference group; c) detecting a quality of the aligned reads and selecting only reads having a quality equal to or higher than a cut-off value; d) segmenting the reference genome into predetermined bins, and detecting and normalizing amounts of the selected reads in the respective bins; e) calculating a mean and a standard deviation of normalized reads matched to each bin of the reference group and then calculating a Z score from normalized values in step d); f) segmenting chromosome using the Z score and calculating an I score; g) obtaining breast tissue image reading information after chemotherapy; and h) determining that a prognosis of breast cancer is bad when the resulting I score is equal to or higher than a cut-off value and the read breast tissue image information is positive.

In another aspect, the present invention is directed to a method of providing information for determining the prognosis of breast cancer including the method.

In the present invention, the breast cancer may be any type of cancer that occurs in the breast and more specifically includes ductal carcinoma in situ, inflammatory carcinoma in situ, invasive ductal carcinoma, and invasive lobular carcinoma, non-invasive ductal carcinoma and non-invasive lobular carcinoma, but is not limited thereto.

As used herein, the term "prognosis" means the prediction of the progression of cancer, recurrence of cancer and/or the possibility of metastasis of cancer. The prediction method of the present invention can be used to make a decision on clinical treatment by selecting the most appropriate treatment method for any particular patient. The prediction method of the present invention is a valuable tool for diagnosis regarding the determination as to whether or not the progression of cancer, recurrence of cancer and/or the possibility of metastasis of cancer of a patient are likely to occur, and/or for assisting in diagnosis.

In another aspect, the method according to the present disclosure may be implemented using a computer. In one embodiment, the computer includes one or more processors coupled to a chipset. In addition, a memory, a storage device, a keyboard, a graphics adapter, a pointing device, a network adapter and the like are connected to the chipset. In one embodiment, the performance of the chipset is acquired by a memory controller hub and an I/O controller hub. In another embodiment, the memory may be directly coupled to a processor instead of the chipset. The storage device is any device capable of maintaining data, including a hard drive, compact disc read-only memory (CD-ROM), DVD, or other memory devices. The memory is concerned with data and instructions used by the processor. The pointing device may be a mouse, track ball or other type of pointing device, and is used in combination with a keyboard to transmit input data to a computer system. The graphics adapter presents images and other information on a display. The network adapter is connected to the computer system through a local area network or a long-distance communication network. However, the computer used herein is not limited to the above configuration, may not have some configurations, may further include additional configurations, and may also be part of a storage area network (SAN), and the computer of the present invention may be configured to be suitable for the execution of modules in the program for the implementation of the method according to the present invention.

The module used herein may mean a functional and structural combination of hardware to implement the technical idea according to the present invention and software to drive the hardware. For example, it is apparent to those skilled in the art that the module may mean a logical unit of predetermined code and a hardware resource to execute the predetermined code, and does not necessarily mean a physically connected code or one type of hardware.

Example

Hereinafter, the present invention will be described in more detail with reference to examples. However, it will be obvious to those skilled in the art that these examples are provided only for illustration of the present invention, and should not be construed as limiting the scope of the present invention.

Example 1. Calculation of I-score in breast cancer patients and normal subjects

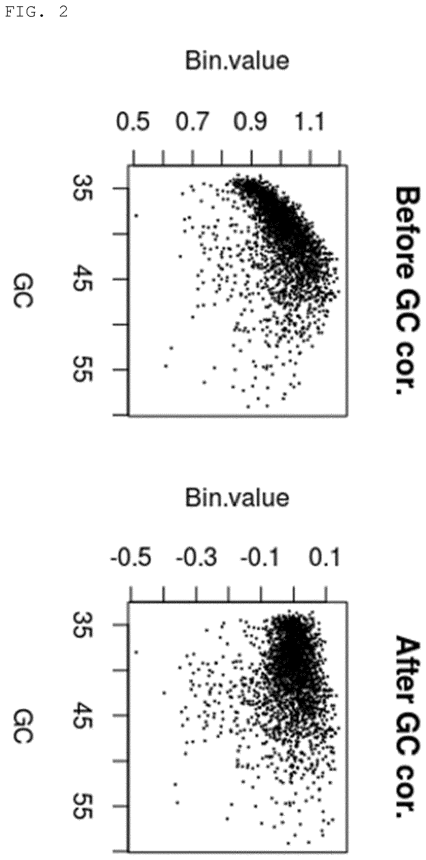

The I-score was calculated using the method described in Korean Patent No. 10-2019-0019315.
More specifically, cell-free DNA was extracted from the plasma samples before anticancer treatment of 456 breast cancer patients in the PEARLY clinical trial (NCT02441933) and received anticancer treatment (neoadjuvant therapy) and underwent surgery, and from the plasma samples of 20 normal people and a library of full-length chromosomes was produced. The extraction of cell-free DNA was performed in the following process: 1) Separation of supernatant (plasma) by sequential centrifugation at 1,600g for 10 minutes and 3,000g for 10 minutes within 4 hours after collection of blood in an EDTA Tube; 2) extraction of cell-free DNA from 0.6 ml of the separated plasma using a plasma circulating DNA kit (Tiangen, China); and 3) reaction of the final extracted cell-free DNA with a Qubit 2.0 Fluorometer and measurement of the concentration (ng/μl), wherein the library was prepared using a MGIEasey Cell-free DNA library Prep Kit (MGI, China), and a total of 2 to 6 ng of cell-free DNA was used for the reaction.
The completed library was subjected to sequencing with a DNBSEQ-G400 sequencer (MGI), and sequence information data corresponding to a mean of 17 million reads per sample was produced.
The Bcl file (including nucleotide sequence information) was converted to fastq format using the next-generation nucleotide sequencing (NGS) equipment, and the library sequence of the fastq file was aligned based on the reference genome Hg19 sequence using the BWA-mem algorithm. It was found that the mapping quality score satisfied 60.
It was confirmed that the distribution of the number of sequencing reads in each chromosome locus bin was biased according to the amount of GC (FIG. 2), and the number of library sequences aligned according to the GC ratio in each chromosome was calibrated using regression analysis.
Then, the Z score was calculated using the following Equation 1:

[Equation 1]

Z score

$$= \frac{\text{Read value of sequence information sample of biological specimen} - \text{Mean sequence information read value of reference group}}{\text{Standard deviation of mean sequence information read value of reference group}}$$

In order to calculate the I-score, first, chromosome was segmented using the CBS algorithm using the calculated Z score in each bin as data.
Then, the I-score was calculated through the following steps:

i) a Z score of each chromosome segment as a mean of Z cores calculated in respective bins included in the segment was calculated;

ii) local regression analysis (LOESS) was performed on each bin to calculate the smoothed Z score (Zn), wherein $n \in \{1,...,N\}$ in which N is the total number of bins;

iii) n_score associated with noise was calculated in accordance with the following Equation 2:

Equation 2: $n_{score} = mean(|B_{n+1} - B_n|)$

wherein **$B_n$ = non smoothed binZscore,** which means the Z score of each bin calculated in step i);

(f-v) calculating I-score in accordance with the following Equation 3:

$$\text{Equation 3: } Iscore = \log\{\sum_{n=1}^{N}(|Z_n \times S_n|)\} - n\_score$$

wherein $S_n$ = *segment Zscore of bin$_n$* which means the Z score of each segment calculated in step i).

Example 2. Confirmation of effect of I-score on progression and survival rate of breast cancer

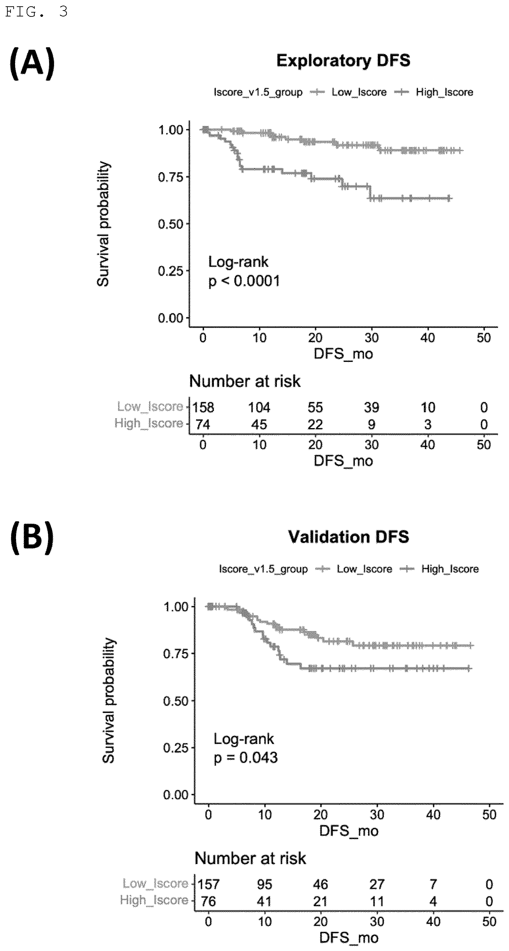

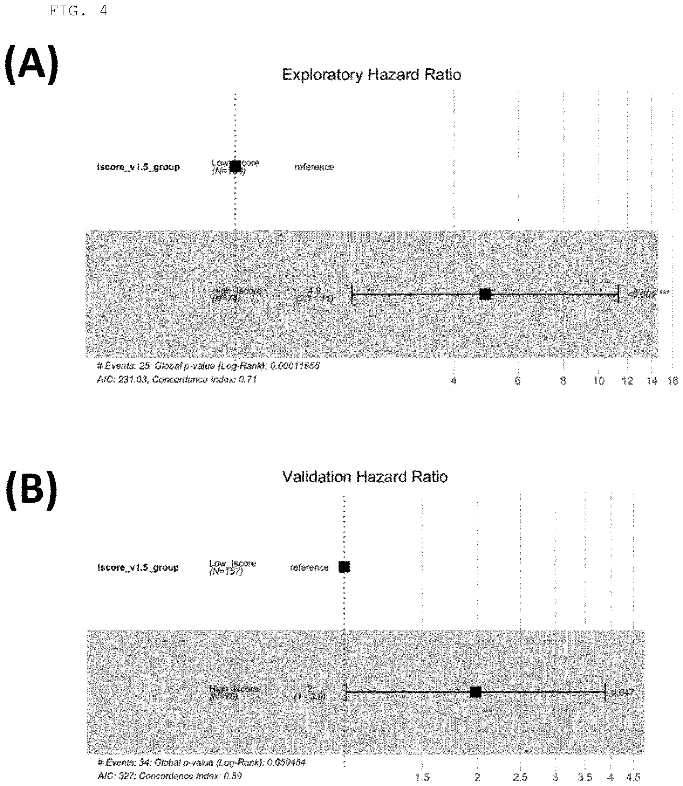

The breast cancer patients in Example 1 were divided into an exploratory group of 232 patients and a validation group of 233 patients and then the correlation between the I score and disease-free survival (DFS) in the exploratory group was analyzed using univariate Cox regression and maximal log-rank test. The result of analysis shows that the group with an I score of 7.81 or higher exhibited greatly reduced DFS and the hazard ratio (HR) for the symptom-free period of the disease increased (FIGS. 3A and 4A). In addition, the validation group exhibited the same results (FIGS. 3B and 4B).

[Table 1]

| | | Exploratory cohort (n = 232) | Validation cohort (n = 233) | Total (n = 465) | p-value |
|---|---|---|---|---|---|
| Age | Mean (±SD) | 48.1 (±10.4) | 48.6 (±10.6) | 48.4 (±10.5) | 0.712* |
| I-score | Median (IQR) | 6.42 (5.27-8.50) | 6.29 (5.36-8.58) | 6.36 (5.34-8.58) | 0.611* |
| I-score | Low | 188 (81%) | 178 (76.4%) | 366 (78.7%) | 0.868 |
| | High | 44 (19%) | 55 (23.6%) | 99 (21.3%) | |
| Clinical T Stage | T1 | 16 (6.9%) | 24 (10.3%) | 40 (8.6%) | 0.543** |
| | T2 | 178 (76.7%) | 177 (76%) | 355 (76.3%) | |
| | T3 | 33 (14.2%) | 27 (11.6%) | 60 (12.9%) | |
| | T4 | 5 (2.2%) | 5 (2.1%) | 10 (2.2%) | |
| Clinical N Stage | N0 | 79 (34.1 %) | 100 (42.9%) | 179 (38.5%) | 0.177 |
| | N1 | 100 (43.1%) | 83 (35.6%) | 183 (39.4%) | |
| | N2 | 25 (10.8%) | 28 (12%) | 53 (11.4%) | |
| | N3 | 28 (12.1%) | 22 (9.4%) | 50 (10.8%) | |
| yp T Stage | T0 | 101 (43.5%) | 106 (45.5%) | 207 (44.5%) | 0.409** |
| | T1 | 83 (35.8%) | 89 (38.2%) | 172 (37%) | |
| | T2 | 28 (12.1%) | 25 (10.7%) | 53 (11.4%) | |
| | T3 | 4 (1.7%) | 6 (2.6%) | 10 (2.2%) | |
| | T4 | 2 (0.9%) | 2 (0.9%) | 4 (0.9%) | |
| | Tis | 14 (6%) | 5 (2.1%) | 19 (4.1%) | |
| yp N Stage | N0 | 183 (78.9%) | 177 (76%) | 360 (77.4%) | 0.674** |
| | N1 | 33 (14.2%) | 31 (13.3%) | 64 (13.8%) | |
| | N1mi | 5 (2.2%) | 9 (3.9%) | 14 (3%) | |
| | N2 | 8 (3.4%) | 9 (3.9%) | 17 (3.7%) | |
| | N3 | 3 (1.3%) | 6 (2.6%) | 9 (1.9%) | |
| | N4 | 0 (0%) | 1 (0.4%) | 1 (0.2%) | |
| pCR | No | 120 (51.7%) | 124 (53.2%) | 244 (52.5%) | 0.747 |

(continued)

| | | Exploratory cohort (n = 232) | Validation cohort (n = 233) | Total (n = 465) | p-value |
|---|---|---|---|---|---|
| | **Yes** | 112 (48.3%) | 109 (46.8%) | 221 (47.5%) | |

Abbreviations: IQR, interquartile range; SD, standard deviation; pCR, pathologic complete response; CNA, copy number aberration
*calculated by *t*-test
**calculated Fisher's exact test

Example 3. Confirmation of correlation between I-score and pCR

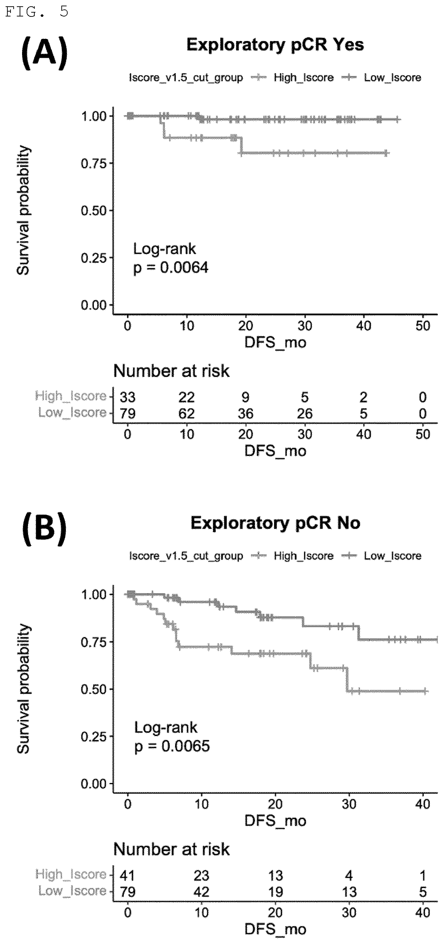

The relationship between the presence of pathological complete response, which is a strong factor in predicting breast cancer prognosis, and the I score, was determined using multivariate Cox analysis. The result showed that, as shown in FIG. 5, the DFS of the exploratory group decreased when the I score was equal to or higher than the reference value regardless of pCR.

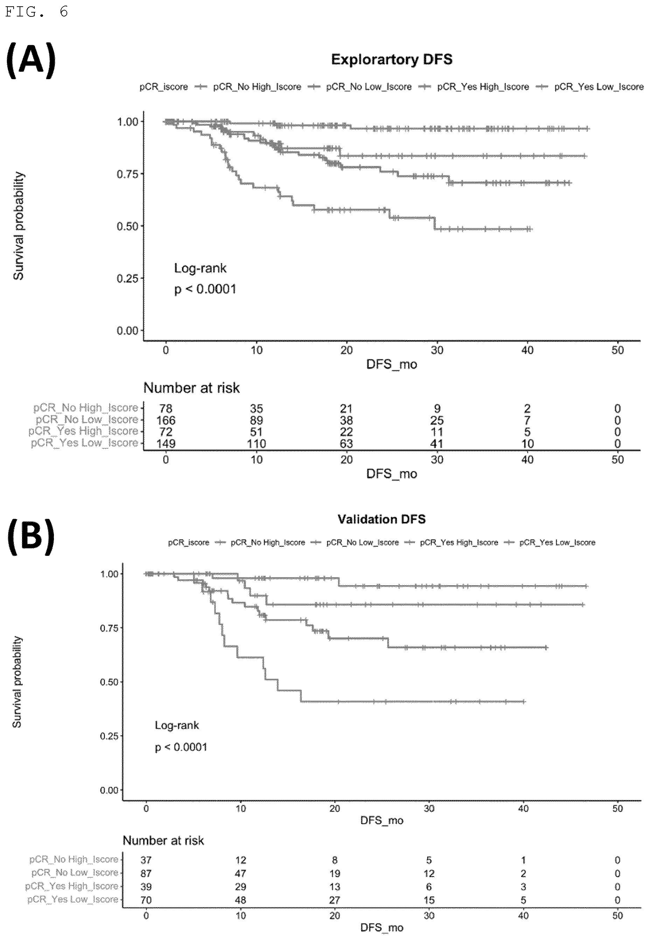

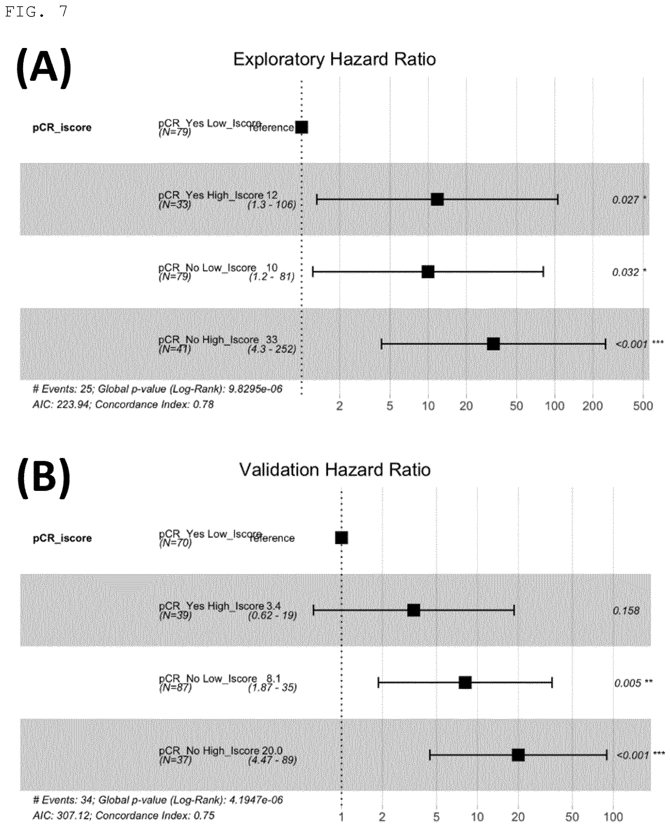

In addition, it can be seen that, since pCR and I score each act as independent prognostic predictors, more detailed prognosis prediction is possible when the patients were divided into four groups depending on a combination of pCR and I (FIGS. 6 and 7).

The four groups are as follows:

(1) Group with I score equal to or higher than the reference value and negative pCR

(2) Group with I score equal to or higher than the reference value and positive pCR

(3) Group with I score lower than the reference value and negative pCR

(4) Group with I score lower than the reference value and positive pCR

As can be seen from FIGS. 6 and 7, Group 1 had the worst prognosis and Group 4 had the best prognosis.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

[Industrial applicability]

The method for determining the prognosis of breast cancer according to the present invention uses next-generation sequencing (NGS) and thereby is capable of improving the accuracy of prognostic prediction of breast cancer patients, as well as the accuracy of prognostic prediction based on cell-free DNA with a very low concentration, which has conventionally been difficult to detect, and of increasing commercial applicability. Therefore, the method of the present invention is useful for determining the prognosis of breast cancer patients.

**Claims**

1. A method of predicting a prognosis of breast cancer based on cell-free DNA (cfDNA), the method comprising:

   a) obtaining reads (sequence information) of the cell-free DNA isolated from a biological sample before chemotherapy;
   b) aligning the reads to a reference genome database of a reference group;
   c) detecting a quality of the aligned reads and selecting only reads having a quality equal to or higher than a cut-off value;
   d) segmenting the reference genome into predetermined bins, and detecting and normalizing amounts of the selected reads in the respective bins;
   e) calculating a mean and a standard deviation of normalized reads matched to each bin of the reference group and then calculating a Z score from normalized values in step d) ;

f) segmenting chromosome using the Z score and calculating an I score;

g) obtaining breast tissue image reading information after chemotherapy; and

h) determining that a prognosis of breast cancer is bad when the resulting I score is equal to or higher than a cut-off value and the read breast tissue image information is positive.

2. The method according to claim 1, wherein step a) is carried out by a process comprising:

(a-i) removing proteins, fats and other residues from the isolated cell-free DNA using a salting-out method, a column chromatography method, or a bead method to obtain purified nucleic acids;

(a-ii) producing a single-end-sequencing or paired-end-sequencing library from the purified nucleic acids;

(a-iii) applying the produced library to a next-generation sequencer; and

(a-iv) obtaining reads of the nucleic acids from the next-generation sequencer.

3. The method according to claim 2, further comprising:

between the steps (a-i) and (a-ii), randomly fragmenting the nucleic acids purified in the step (a-i) by an enzymatic digestion, pulverization or HydroShear method to produce the single-end sequencing or paired-end sequencing library.

4. The method according to claim 1, wherein step a) of obtaining the reads comprises obtaining the isolated cell-free DNA through full-length genome sequencing with a depth of 0.01 to 100 reads.

5. The method according to claim 1, wherein step c) is carried out through a process comprising:

(c-i) specifying a region of each aligned nucleic acid sequence; and

(c-ii) selecting a sequence satisfying a cut-off value of a mapping quality score and a cut-off value of a GC ratio within the region.

6. The method according to claim 5, wherein the cut-off value of the mapping quality score is 15 to 70 and the cut-off value of the GC ratio is 30 to 60%.

7. The method according to claim 5, wherein step c) is performed excluding data of a centromere or a telomere of the chromosome.

8. The method according to claim 1, wherein step d) is carried out through a process comprising:

(d-i) segmenting the reference genome into predetermined bins;

(d-ii) calculating a number of reads aligned in each bin and an amount of GC of the reads;

(d-iii) performing a regression analysis based on the number of reads and the amount of GC to calculate a regression coefficient; and

(d-iv) normalizing the number of reads using the regression coefficient.

9. The method according to claim 8, wherein the predetermined bin in step (d-i) is 100 kb to 2 Mb in length.

10. The method according to claim 1, wherein step e) of the calculation is carried out using Equation 1 below.

[Equation 1]

$$Z \ score = \frac{\text{Read value of sequence information sample of biological specimen} - \text{Mean sequence information read value of reference group}}{\text{Standard deviation of mean sequence information read value of reference group}}$$

11. The method according to claim 1, wherein step (f) is carried out by a process comprising:

(f-i) segmenting a chromosome region using circular binary segmentation (CBS) based on a Z score in each bin;

(f-ii) obtaining a Z score of each chromosome segment as a mean of Z cores calculated in respective bins included in the segment;

(f-iii) calculating the smoothed Z score (Zn) by performing local regression analysis (LOESS) on each bin, wherein $n \in \{1,...,N\}$ in which N is the total number of bins;

(f-iv) calculating n_score associated with noise in accordance with the following Equation 2:

$$\text{Equation 2:} \quad n_{score} = mean(|B_{n+1} - B_n|)$$

wherein $B_n$ = *non smoothed binZscore,* which means the Z score of each bin calculated in step i); and

(f-v) calculating I-score in accordance with the following Equation 3:

$$\text{Equation 3:} \quad \text{Iscore} = \log\{\textstyle\sum_{n=1}^{N}(|Z_n \times S_n|)\} - n\_score$$

wherein $S_n$ = *segment Zscore of bin_n* which means the Z score of each segment calculated in step i).

12. The method according to claim 1, wherein the breast tissue image is selected from the group consisting of a histochemical-stain breast tissue sample image, and a fluorescent stain breast tissue sample image.

13. The method according to claim 1, wherein the positive breast tissue image reading information means that cancer cells are identified in the image.

14. The method according to claim 1, wherein the cut-off value of the I score is 5 to 10.

15. The method according to claim 1, further comprising classifying a case where the I score is equal to or higher than a cut-off value and the read image information is negative as a moderate risk group, classifying a case where the I score is lower than a cut-off value and the read image information is positive, as a high risk group, and classifying a case where the I score is equal to or higher than a cut-off value and the read image information is positive as an ultra-high risk group.

16. A method of providing information for determining a prognosis of breast cancer using the method according to any one of claims 1 to 15.

17. A method of determining a prognosis of breast cancer comprising predicting a prognosis of breast cancer using the method according to any one of claims 1 to 15.

18. A device for predicting a prognosis of breast cancer based on cell-free DNA (cfDNA), the device comprising:

    a decoder for decoding reads (sequence information) of cell-free DNA isolated from a biological sample before chemotherapy;
    an aligner for aligning the decoded reads to a reference genome database of a reference group;
    a quality controller for selecting only reads having a quality equal to or higher than a cut-off value from the aligned reads;
    an I score calculator for calculating a Z score of the selected sequence information (reads) by comparison with a reference group sample and then calculating an I score (I-score) based thereon;
    a read image information receiver for obtaining breast tissue image reading information after chemotherapy; and
    a determiner for determining that the prognosis of breast cancer is bad when the I score is equal to or higher than a cut-off value and the read image information is positive.

19. A computer-readable medium comprising an instruction configured to be executed by a processor for determining a prognosis of breast cancer, the computer-readable medium comprising:

    a) obtaining reads (sequence information) of cell-free DNA isolated from a biological sample before chemotherapy;
    b) aligning the reads to a reference genome database of a reference group;
    c) detecting a quality of the aligned reads and selecting only reads having a quality equal to or higher than a cut-off value;

d) segmenting the reference genome into predetermined bins, and detecting and normalizing amounts of the selected reads in the respective bins;

e) calculating a mean and a standard deviation of normalized reads matched to each bin of the reference group and then calculating a Z score from normalized values in step d) ;

f) segmenting chromosome using the Z score and calculating an I score;

g) obtaining breast tissue image reading information after chemotherapy; and

h) determining that a prognosis of breast cancer is bad when the resulting I score is equal to or higher than a cut-off value and the read breast tissue image information is positive.

FIG. 1

```
┌─────────────────────────────────────────┐
│  Extracting cell-free nucleic acid from  │
│                 plasma                    │
└─────────────────────────────────────────┘
                    │
                    ▼
┌─────────────────────────────────────────┐
│  Obtaining nucleic acid fragments by      │
│      massive parallel sequencing          │
└─────────────────────────────────────────┘
                    │
                    ▼
┌─────────────────────────────────────────┐
│  Aligning fragment data to human          │
│      reference chromosomes                │
└─────────────────────────────────────────┘
                    │
                    ▼
┌─────────────────────────────────────────┐
│      Checking quality of aligned data     │
└─────────────────────────────────────────┘
                    │
                    ▼
┌─────────────────────────────────────────┐
│          Calculating I-score              │
└─────────────────────────────────────────┘
                    │
                    ▼
┌─────────────────────────────────────────┐
│  Determining prognosis using I-score      │
│               and pCR                     │
└─────────────────────────────────────────┘
```

FIG. 2

FIG. 3

**(A)**

Exploratory DFS

**(B)**

Validation DFS

FIG. 4

**(A)**

Exploratory Hazard Ratio

**(B)**

Validation Hazard Ratio

FIG. 5

(A) Exploratory pCR Yes

(B) Exploratory pCR No

FIG. 6

**(A)**

Explorartory DFS

**(B)**

Validation DFS

FIG. 7

(A) Exploratory Hazard Ratio

(B) Validation Hazard Ratio

## INTERNATIONAL SEARCH REPORT

| International application No. |
| --- |
| **PCT/KR2022/019625** |

| A. | CLASSIFICATION OF SUBJECT MATTER |
| --- | --- |

**G16H 50/50**(2018.01)i; **G16H 20/40**(2018.01)i; **G16H 50/20**(2018.01)i; **G16B 30/10**(2019.01)i; **G16B 50/00**(2019.01)i; **G16H 30/40**(2018.01)i; **C12Q 1/6886**(2018.01)i; **G01N 33/483**(2006.01)i; **G06T 7/00**(2006.01)i; **G06F 17/18**(2006.01)i

According to International Patent Classification (IPC) or to both national classification and IPC

| B. | FIELDS SEARCHED |
| --- | --- |

Minimum documentation searched (classification system followed by classification symbols)

G16H 50/50(2018.01); C12Q 1/6886(2018.01); G16B 15/30(2019.01); G16B 25/10(2019.01); G16B 30/00(2019.01); G16B 40/20(2019.01); G16B 50/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 무세포 DNA(cell free DNA), 유방암(breast cancer), 서열정보(reads), Z점수(Z score), I점수(I score), 예후(prognosis), 예측(prediction)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2019-0085667 A (GREEN CROSS GENOME CORPORATION) 19 July 2019 (2019-07-19)<br>See paragraph [0116], claims 1-9 and figure 1. | 1-19 |
| A | PANAGOPOULOU, Maria et al. Circulating Cell-Free DNA in Breast Cancer: Searching for Hidden Information towards Precision Medicine. Cancers. Vol. 13, No. 728, 10 February 2021.<br>See entire document. | 1-19 |
| A | US 2019-0264291 A1 (THE CHINESE UNIVERSITY OF HONG KONG) 29 August 2019 (2019-08-29)<br>See entire document. | 1-19 |
| A | KR 10-2021-0073526 A (FREENOME HOLDINGS, INC.) 18 June 2021 (2021-06-18)<br>See entire document. | 1-19 |
| A | KR 10-2021-0034363 A (KOREA UNIVERSITY RESEARCH AND BUSINESS FOUNDATION et al.) 30 March 2021 (2021-03-30)<br>See entire document. | 1-19 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 March 2023** | **15 March 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2022/019625**

| Patent document cited in search report | | | Publication date (day/month/year) | Patent family member(s) | | | Publication date (day/month/year) |
|---|---|---|---|---|---|---|---|
| KR 10-2019-0085667 | | A | 19 July 2019 | KR | 10-2029393 | B1 | 07 October 2019 |
| | | | | WO | 2019-139363 | A1 | 18 July 2019 |
| US 2019-0264291 | | A1 | 29 August 2019 | AU | 2013-278994 | A1 | 29 January 2015 |
| | | | | AU | 2013-278994 | B2 | 06 April 2017 |
| | | | | AU | 2013-278994 | C1 | 12 December 2019 |
| | | | | AU | 2017-204558 | A1 | 27 July 2017 |
| | | | | AU | 2017-204558 | B2 | 10 October 2019 |
| | | | | AU | 2020-200122 | A1 | 30 January 2020 |
| | | | | AU | 2020-200122 | B2 | 19 May 2022 |
| | | | | AU | 2022-209294 | A1 | 25 August 2022 |
| | | | | CA | 2876327 | A1 | 27 December 2013 |
| | | | | CA | 2876327 | C | 29 September 2020 |
| | | | | CA | 3080937 | A1 | 27 December 2013 |
| | | | | CN | 104662168 | A | 27 May 2015 |
| | | | | CN | 104662168 | B | 05 December 2017 |
| | | | | CN | 107779506 | A | 09 March 2018 |
| | | | | CN | 107779506 | B | 15 July 2022 |
| | | | | CN | 113151474 | A | 23 July 2021 |
| | | | | DK | 2864501 | T3 | 05 November 2018 |
| | | | | DK | 3456843 | T3 | 25 October 2021 |
| | | | | EA | 037292 | B1 | 05 March 2021 |
| | | | | EA | 201500027 | A1 | 29 May 2015 |
| | | | | EA | 202092900 | A2 | 31 March 2021 |
| | | | | EA | 202092900 | A3 | 30 June 2021 |
| | | | | EP | 2864501 | A2 | 29 April 2015 |
| | | | | EP | 2864501 | A4 | 16 March 2016 |
| | | | | EP | 2864501 | B1 | 08 August 2018 |
| | | | | EP | 3456843 | A1 | 20 March 2019 |
| | | | | EP | 3456843 | B1 | 28 July 2021 |
| | | | | EP | 3919627 | A1 | 08 December 2021 |
| | | | | EP | 3919627 | A4 | 08 December 2021 |
| | | | | ES | 2687847 | T3 | 29 October 2018 |
| | | | | ES | 2894479 | T3 | 14 February 2022 |
| | | | | HK | 1204013 | A1 | 06 November 2015 |
| | | | | HK | 1205533 | A1 | 18 December 2015 |
| | | | | HK | 1246830 | A1 | 14 September 2018 |
| | | | | HU | E056915 | T2 | 28 March 2022 |
| | | | | IL | 235967 | A | 26 September 2019 |
| | | | | IL | 235967 | B | 26 September 2019 |
| | | | | IL | 268645 | A | 31 October 2019 |
| | | | | IL | 278867 | A | 31 January 2021 |
| | | | | JP | 2015-527057 | A | 17 September 2015 |
| | | | | JP | 2018-191640 | A | 06 December 2018 |
| | | | | JP | 2021-191280 | A | 16 December 2021 |
| | | | | JP | 6371280 | B2 | 08 August 2018 |
| | | | | JP | 6930948 | B2 | 01 September 2021 |
| | | | | KR | 10-1884909 | B1 | 02 August 2018 |
| | | | | KR | 10-2015-0032708 | A | 27 March 2015 |
| | | | | KR | 10-2018-0088922 | A | 07 August 2018 |
| | | | | KR | 10-2020-0035507 | A | 03 April 2020 |

Form PCT/ISA/210 (patent family annex) (July 2022)

**INTERNATIONAL SEARCH REPORT**
Information on patent family members

International application No.

**PCT/KR2022/019625**

| Patent document cited in search report | | | Publication date (day/month/year) | Patent family member(s) | | | Publication date (day/month/year) |
|---|---|---|---|---|---|---|---|
| | | | | KR | 10-2021-0040464 | A | 13 April 2021 |
| | | | | KR | 10-2022-0038181 | A | 25 March 2022 |
| | | | | KR | 10-2096611 | B1 | 02 April 2020 |
| | | | | KR | 10-2237923 | B1 | 08 April 2021 |
| | | | | KR | 10-2375645 | B1 | 17 March 2022 |
| | | | | MX | 2014016058 | A | 10 April 2015 |
| | | | | MX | 360264 | B | 26 October 2018 |
| | | | | PT | 3456843 | T | 26 October 2021 |
| | | | | SG | 10201808217 | A | 30 October 2018 |
| | | | | SG | 11201408113 | A | 29 January 2015 |
| | | | | TW | 201403066 | A | 16 January 2014 |
| | | | | TW | 201920952 | A | 01 June 2019 |
| | | | | TW | 202122796 | A | 16 June 2021 |
| | | | | TW | I636255 | B | 21 September 2018 |
| | | | | US | 11261494 | B2 | 01 March 2022 |
| | | | | US | 2014-0100121 | A1 | 10 April 2014 |
| | | | | US | 2018-0202003 | A1 | 19 July 2018 |
| | | | | WO | 2013-190441 | A2 | 27 December 2013 |
| | | | | WO | 2013-190441 | A3 | 27 February 2014 |
| | | | | ZA | 201409281 | B | 30 August 2017 |
| KR | 10-2021-0073526 | A | 18 June 2021 | AU | 2019-356497 | A1 | 18 March 2021 |
| | | | | CA | 3107948 | A1 | 16 April 2020 |
| | | | | CN | 112740239 | A | 30 April 2021 |
| | | | | EP | 3815005 | A1 | 05 May 2021 |
| | | | | JP | 2022-511243 | A | 31 January 2022 |
| | | | | SG | 11202100960 | A | 25 February 2021 |
| | | | | US | 2021-0272653 | A1 | 02 September 2021 |
| | | | | WO | 2020-076772 | A1 | 16 April 2020 |
| KR | 10-2021-0034363 | A | 30 March 2021 | US | 2022-0401077 | A1 | 22 December 2022 |
| | | | | WO | 2021-054752 | A2 | 25 March 2021 |
| | | | | WO | 2021-054752 | A3 | 22 July 2021 |

Form PCT/ISA/210 (patent family annex) (July 2022)

**REFERENCES CITED IN THE DESCRIPTION**

**Patent documents cited in the description**

- KR 1020190019315

**Non-patent literature cited in the description**

- **LEARY R. J. et al.** *Sci. Transl. Med.,* 2012, vol. 4 (162
- **STOVER DG. et al.** *J. Clin. Oncol.,* vol. 36 (6), 543-553